# EUROPEAN PATENT APPLICATION

(11) **EP 1 894 916 A2**
(43) Date of publication of application: **05.03.2008**
(21) Application number: 06769557.7
(22) Date of filing: 23.06.2006
(51) Int. Cl.: C07C 259/06, A61K 31/16, A61P 35/04

(54) **DICARBONIC ACID DERIVATIVES, METASTASIS INHIBITORS AND AGENTS INCREASING CHEMOTHERAPEUTIC ACTIVITY OF ANTI-TUMOR PREPARATIONS, METHOD FOR ENHANCING THE EFFICIENCY OF CYTOSTATICS AND METASTASIS PROCESS INHIBITING METHOD**

(30) Priority: 24.06.2005 RU 2005119520
(71) Applicant: Institut Problem Khimicheskoi Fiziki Rossiiskoi AK, Moskovskaya obl., 142432 (RU)
(72) Inventor: ALDOSHIN, Sergei Mikhailovich, Moskovskaya obl., 142432 (RU); KONOVALOVA, Nina Petrovna, Moskovskaya obl., 142432 (RU); LARUKOVA, Marina Victorovna, Moskovskaya obl., 142400 (RU); SASHENKOVA, Tatyana Evgenievna, Moskovskaya obl., 142432 (RU); FADEEV Mikhail Arsenievich, Moskovskaya obl., 142432 (RU); FEDOROV, Boris Sergeevich, Moskovskaya obl., 142432 (RU)
(74) Representative: Henriksson, Dan Ragnar Mikael
(86) International application number: PCT/RU2006/000330
(87) International publication number: WO 2007/001211

(57) **Abstract**

The invention relates to dicarbonic acid derivatives and concerns dicarbonic acid monooxydamines and dioxydamides, which are physiologically active substances and can be used as low-toxic and non-toxic agents for enhancing the anti-tumor and anti-metastasis effects of known cytostatics (cyclophosphane and cisplatin) in cytostatic chemotherapy of tumors.

The object of the present invention is to broaden the assortment of agents affecting a live organism in order to enhance anti-tumor and anti-metastasis effects of known cytostatics.

This object is resolved by the properties of dicarbonic acid monooxydamines and dioxydamides.

The essence of the invention consists in that dicarbonic acid derivatives, the formulas of which are presented in the specification, are proposed as agents for enhancing anti-tumor and anti-metastasis effects of cytostatics.

The claimed compounds can be used in medical practice as low-toxic agents for enhancing anti-tumor and anti-metastasis effects of known cytostatics with a simultaneous 4-fold reduction of the therapeutic dose of cytostatics. The use of the proposed compounds in a combination with cisplatin at the minimum doses makes it possible to completely inhibit the metastasis process in experimental B-16 melanoma. Furthermore, the use of the proposed compounds at the minimum doses in a combination with the minimum doses of cytostatics (when neither of the preparations alone exhibitsefficacy) makes it possible to obtain a high therapeutic effect in treating leukemias (survival of animals with P-388 leukemia in the test group is 100%).

## Description

The invention relates to dicarbonic acid derivatives which are physiologically active substances and can be used in medical practice as low-toxic agents for enhancing anti-tumor and anti-metastasis activity of known cytostatics in cytostatic chemotherapy of tumors. The present invention specifically relates to dicarbonic acid mono- and dioxyamides of general formula 2:

RCONHOH (2)

wherein R is HONHCO; HONHCOCH(OH)CH(OH); HOOCCH₂CH₂;
HOOCCH=CH.

Compounds of formula 2 are physiologically active substances and can be used as agents for enhancing anti-tumor and anti-metastasis effects of cytostatics. Furthermore, unlike xenobiotics, these compounds do not cause the development of a complex of intricate reactions to neutralize the latter using the significant cellular energetic resources [Saprin A.N. Fermenty metabolizma i detoksikatsii xenobioticov (Metabolism and detoxication enzymes of xenobiotics). Uspekhi Biologicheskoj Khimii (Advances in Biological Chemistry) 1991, vol.32, p.146-175].

It is known that cisplatin and cyclophosphane are used alone or in combination with other cytostatics or as medicaments in treating oncological diseases [Protivoopukholevaya khimioterapiya (Anti-tumor Chemotherapy). Edited by N.I.Perevozchikova, a reference book, Moscow, "Meditsina" publisher 1986. N.P. Konovalova, S.A. Goncharova, L.M. Volkova, T.A. Raevskaya, L.T. Eremenko and A.M. Korolev. "Donor oxida azota povyshajet effectivnost' cytostaticheskoj terapii i zaderzhivajet razvitije lekarstvennoj rezistantnosti (Nitrogen oxide donor enhances efficiency of cytostatic therapy and delays development of drug resistance)", 2003, vol. 49, No 1, p. 71-75]. However, as a rule the maximum tolerable doses of cytostatics are used in the chemotherapy of leukemias and some other oncological diseases in order to achieve positive effects, and because of that the cytostatics exhibit a very high toxicity with respect to warm-blooded animals.

The object of the present invention is to develop earlier unknown dicarbonic acid derivatives which possess low toxicity with respect to warm-blooded animals and make it possible to achieve significant therapeutic effects in combined therapy of leukemias and inhibition of metastasis using the minimum doses of the derivatives indicated above in combination with the minimum doses of known cytostatics.

Novel compounds of formula 2 were synthesized according to a known process by treatment of carbonic acid alkyl ethers with hydroxylamine (Ber. 1953, Bd 86, page 1186).

The invention is characterized by the following examples.
**Example 1.** Tartaric acid dihydroxydiamide (+). A solution of 2.69 g (0.015 mole) tartaric acid dimethyl ether (+) in 10 ml absolute methanol was added drop-wise to 4 ml monomolar hydroxylamine solution in absolute methanol while stirring at room temperature and then left at room temperature for 2-3 days. After that a white crystalline precipitate was filtered off and dried. 1.33 g (73.8%) tartaric acid dihydroxydiamide (+), m.p. 170-171°C. ¹H((DMSO-d₆, δ, m.d.,J, Hz) NMR spectrum 4.22 (d.CH); 5.33(d,OH alcohol); 8.75 (br.s, NH); 10.38 (br.s, OH from COOH). Found (%): C 26.18; H 4.16; N 15.25. C₄H₈N₂O₆. Calculated (%): C 26.6; H 4.44; N 15.6.
**Example 2.** Maleic acid monooxamide (FB-13). The reaction was carried out in a manner similar to Example 1. Following conventional treatment, maleic acid monooxamide was obtained with a yield 74%, m.p. 119-120°C. Found (%): C 36.50; H 3.88; N 10.41. C₄H₅NO₄. Calculated (%): C 36.64; H 3.82; N 10.69. ¹H((DMSO-d₆, δ, m.d.) NMR spectrum 10.0 (br.s, 3H, COOH, NHOH); 6.25 (br.s, 2, CH=CH).
**Example 3.** Aspartic acid monooxamide (FB-14). The reaction was carried out in a manner similar to Example 1. Following conventional treatment, aspartic acid monooxamide in the form of a white crystalline substance was obtained from 15 ml monomolar hydroxylamine solution and 0.0075 M aspartic acid methyl ether with a yield 69.5%, m.p. 142-142°C, C₄H₈N₂O₄. Found (%): C 32.42; H 5.6; N 19.00. Calculated: C 32.43; H 5.40; N 18.92. ¹H(DMSO-d₆, δ, m.d.) NMR spectrum 8.35 (br.s, 5H, COOH, NH₂, NHOH); 3.96 (m, 1H, CH), 271 (m, 2H, CH₂).
**Example 4.** Succinic acid monooxamide. The reaction was carried out in a manner similar to Example 1. Following conventional treatment, succinic acid monooxamide was obtained in the form of a white or colorless substance with a yield 70.2%. Following conventional treatment and drying, the substance has m.p. 116-118°C. Yield 64%. ¹H(DMSO-d₆, *δ*, m.d.) NMR spectrum 12.1 s. (br. 1H, OH); 10.4 (s. br., 1H, OH); 10.4 (s. br., 1H, COOH); 8.73 (s. br. 1H, NH); 2.42 (t, 2H, CH₂); 2.20 (t, 2H, CH₂C=O). Found (%): C 35.94; H 5.08; N 7.89. C₄H₇N0₄. Calculated (%): C 36.09; H 5.26; N 10.5.
   An experimental study of the anti-tumor activity of the FB-13 preparation as a chemosensitizer was carried out on tumor models of P388 leukemia and Lewis metastatic lung carcinoma, which can be seen in Examples 5 and 6.
   **Example 5.** Leukemia P388 was intraperitoneally inoculated to hybrid mice BDF₁. The inoculum was 10⁶ leukemic cells in 0.2 ml normal saline. The preparation was administered at a dose 10 mg/kg, cisplatin - at a dose 2 mg/kg on days 1, 3, 5 and 7 post transplantation. The number of cured (surviving for 60 days) animals served as an assessment criterion. When only cisplatin was administered, this index was 66.7%, in the case of combined administration of cisplatin and FB-13, 100% of the animals survived.
**Example 6.** Lewis lung carcinoma was subcutaneously inoculated to hybrid mice BDF₁. The inoculum was 5x10⁶ tumor cells. The combined effect of FB-13 and cisplatin was investigated. The index of metastasis inhibition (IMI in %) in the lungs served as the assessment criterion. When cisplatin was administered alone, IMI was 80%, in combined administration with FB-13, it was 100%.
**Example 7.** Experimental B-16 melanoma was subcutaneously engrafted to hybrid mice BDF₁. The inoculum was 5x10⁶ tumor cells. The combined effect of FB-13 and cisplatin was investigated. The index of metastasis inhibition (IMI in %) in the lungs served as the assessment criterion. When cisplatin was administered alone, IMI was 52%, in combined administration with FB-13, it was 100%. Tests for acute toxicity were carried out in the group of experimental oncology of the IPKhF of the RAS by intraperitoneal administration of the preparations in water to BDF₁ mice. The claimed subject matters were shown to be non-toxic at a dose of 1,000 mg/kg. These data make it possible to attribute the claimed compounds to the category of low-toxic compounds. The use of these compounds at the minimum doses in combination with the minimum doses of cytostatics (when neither of the preparations taken alone provides a therapeutic effect) makes complete inhibition of the metastasis process in experimental B-16 melanoma or Lewis lung carcinoma possible. Furthermore, the use of dicarbonic acid oxyamides as agents for enhancing the anti-tumor effect makes it possible in a combination with the minimum amounts of oxyamide and a cytostatic (a unit dose of cytostatics being 4 times lower than a therapeutic dose) to enhance the efficacy in the treatment of P-388 leukemia (survival rate of the animals is 100%).

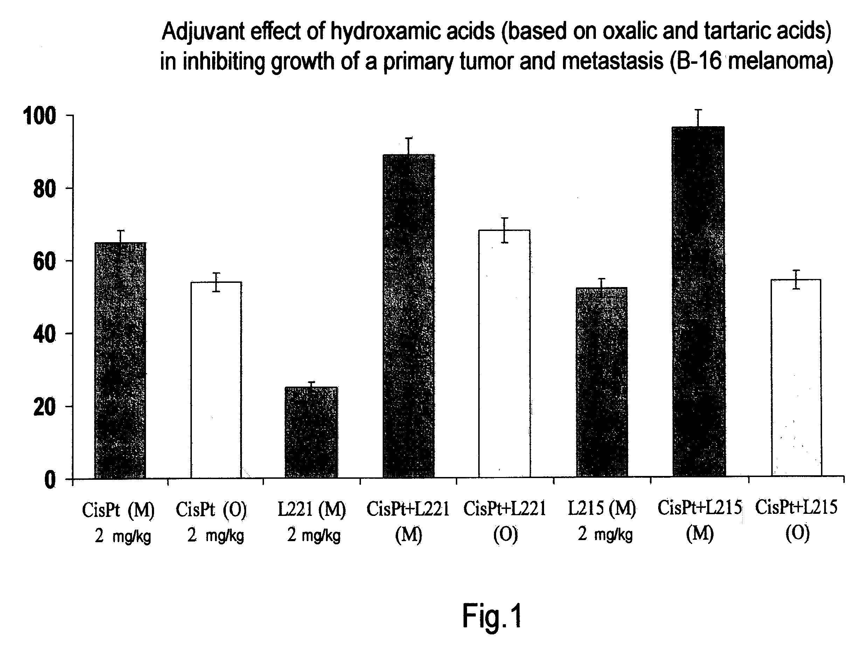

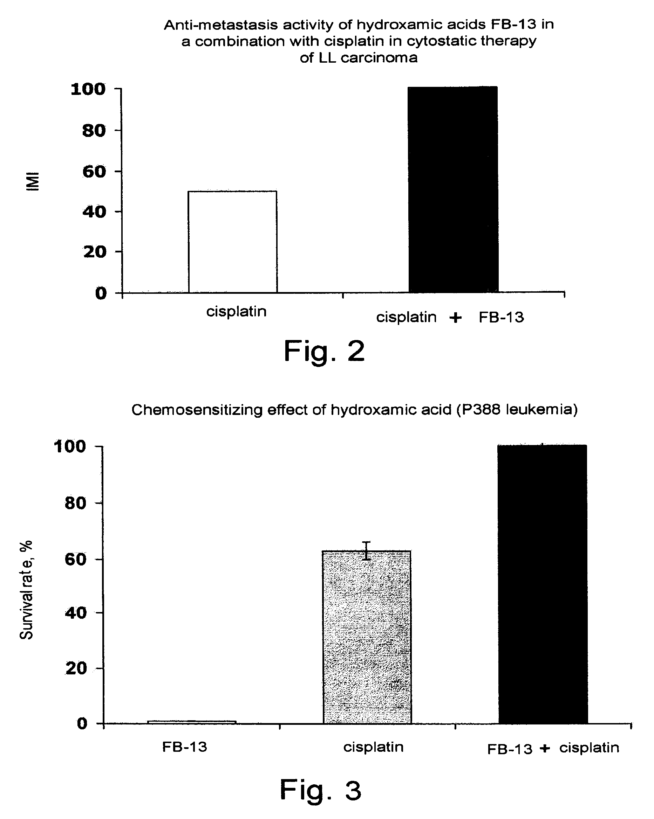

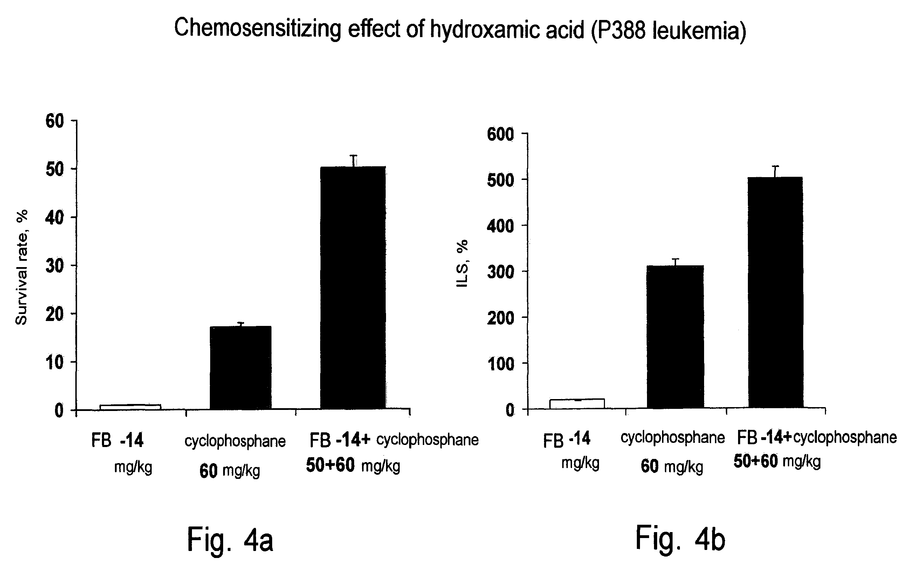

## Claims

1. Use of dicarbonic acids of general formula 2:
RCONHOH (2)
wherein R is HONHCO; HONHCOCH(OH)CH(OH); HOOCCH₂CH₂;
HOOCCH=CH
as metastasis inhibitors and agents enhancing chemotherapeutic activity of anti-tumor preparations.

2. A method for enhancing the efficacy of cytostatics in cytostastic chemotherapy of tumors, **characterized in that** a cytostatic is used in a combination with dicarbonic acid derivatives of formula 2:
RCONHOH (2)
wherein R is HONHCO; HONHCOCH(OH)CH(OH); HOOCCH₂CH₂;
HOOCCH=CH.

3. A method for inhibiting the metastasis process, **characterized in that** a tumor is subjected to the action of a combination of a known cytostatic and dicarbonic acid derivatives of formula 2:
RCONHOH (2)
wherein R is HONHCO; HONHCOCH(OH)CH(OH); HOOCCH₂CH₂;
HOOCCH=CH.
